# EUROPEAN PATENT SPECIFICATION

(11) **EP 4 076 149 B1**
(45) Date of publication and mention of the grant of the patent: **24.12.2025**
(21) Application number: 20830047.5
(22) Date of filing: 21.12.2020
(51) Int. Cl.: A61B 5/00, A61B 5/026, A61B 5/0275

(54) **SYSTEM AND COMPUTER PROGRAM FOR IDENTIFYING BLOOD VESSELS DURING FLUORESCENCE IMAGING**
SYSTEM UND COMPUTERPROGRAMM ZUR IDENTIFIZIERUNG VON BLUTGEFÄSSEN WÄHREND DER FLUORESZENZBILDGEBUNG
SYSTÈME ET PROGRAMME D'ORDINATEUR POUR L'IDENTIFICATION DE VAISSEAUX SANGUINS PENDANT L'IMAGERIE PAR FLUORESCENCE

(30) Priority: 19.12.2019 EP 19218169
(43) Date of publication of application: 26.10.2022
(73) Proprietor: Perfusion Tech ApS, 2100 København Ø (DK)
(72) Inventor: LUND, Morten Toft, 2200 Copenhagen N (DK); MADSEN, Mads Holst Aagaard, 2100 Copenhagen Ø (DK)
(74) Representative: FRKelly
(86) International application number: PCT/EP2020/087507
(87) International publication number: WO 2021/123446

(56) References cited:
- WO-A1-2019/143676
- US-A1- 2017 245 766
- US-A1- 2018 125 378
- US-A1- 2018 158 187
- US-A1- 2019 200 869
- US-B1- 6 351 663

## Description

The present disclosure relates to a system and a method for continuously identifying blood vessels in tissue by means of fluorescence imaging. In particular the present disclosure relates to continuously measuring and assessing hemodynamics in tissue in medical procedures using fluorescence imaging wherein the administration of the fluorescent agent is controlled and automated and thereby identifying, mapping and visualizing blood vessels in the tissue.

### Background

Tissue dissection is a major part of surgery today, often occupying up to 50% of surgical time. Great care is taken to isolate blood vessels and other central structures from the surrounding connective tissue, before the actual surgical procedure is carried out. This is the case in surgical procedures such as bowel resection and anastomosis. But despite this, unintentional tearing of blood vessels occurs frequently and results in excessive bleeding and other complications, prolonging surgical time significantly.

The challenge is that to the naked eye blood vessels and central structures are not readily distinguishable from surrounding connective tissue. And the problem is enhanced further with the increasing prevalence of obesity, as patients with obesity have large amounts of connective tissue, consisting mainly of fat. The surgeons therefore need a tool which can easily distinguish and map the blood vessels in the connective tissue. This will allow for a safer and faster tissue dissection, and has the potential to decrease surgical time substantially. Shorter surgical time is a benefit for both the patient, who is exposed to less surgical stress, and for the hospital as a whole as this will allow for more surgical procedures in the same time span.

Fluorescent imaging is one such tool wherein injection of fluorescence imaging agents, aka fluorescent contrast agents, aka fluorescent agents, such as indocyanine green (ICG), is provided to visualize blood flow and perfusion in tissue of anatomical structures. One recent example is the Leica Microsystems GLOW800 augmented reality (AR) system providing simultaneous white-light and real-time fluorescent blood flow view, such that cerebral anatomy can be observed in natural color, augmented by real-time vascular flow.

US 2018/125378 discloses fluorescence based flow imaging by means of fluorescence based tracking of a light-emitting marker in a bodily fluid stream. Time characteristics of light output of the marker is determined and flow characteristics based on the time characteristics are calculated. Further methods of fluorescence based imaging are known from US-6351663-B1, US-2019/200869-A1, WO-2019/143676-A1, US-2017/245766-A1, and US-2018/158187-A1.

### Summary

The present invention is defined in the appended claims.

One limitation of the known fluorescence imaging procedures is that the typical dose of e.g. ICG used today requires at least 20-30 minutes washout before a new measurement can be made. I.e. each dose of ICG can be seen as a "single image exposure" as the bolus flows through the arteries providing a snapshot of the perfusion situation. The problem is that during bowel surgery the bowel is subject to peristaltic movements and during the tissue dissection the surgeons are slowly working through the connective tissue and a single snapshot for each 30 minutes is therefore hardly usable. To overcome this limitation the present inventors in the PCT application entitled "System and method for automatic perfusion measurement" pending as PCT/EP2019/065648 have presented a novel administration regime for fluorescent imaging that allows for "continuous" perfusion monitoring based on automated administration of time separated micro-boluses of at least one fluorescent agent. This approach creates a fluorescent signal from the perfused tissue that is fluctuating over time with a predetermined pattern.

The present inventors have now realized that the novel dose regime disclosed in PCT/EP2019/065648 can be applied to identify blood vessels, for example blood vessels hidden in connective tissue. One aspect of the present disclosure therefore relates to a (computer implemented) method for identifying blood vessels in tissue of a subject, e.g. during a medical procedure. The method comprises the step of continuously generating and/or receiving a fluorescent signal from (blood vessels in) the tissue wherein the intensity and/or the wavelength of the fluorescent signal is varying and/or oscillating in a predetermined way, e.g. oscillating in a predetermined pattern, i.e. the oscillating pattern is preferably imposed externally in a predetermined and controllable way. The oscillating pattern is preferably generated from a series of small boluses of at least one fluorescent imaging agent, i.e. the oscillating pattern is originating from the repeated bolus injections. The oscillating pattern can be seen as having a frequency (aka period) such that a series of boluses administered with a predefined and/or controlled duration between subsequent boluses determines the frequency of the pattern.

Inherent time differences in each of the fluorescent signals originating from each small bolus due to the subject specific hemodynamiscs can be used to identify blood vessels. I.e. the presently disclosed approach may comprises the step of analysing at least part of the fluorescence images and determining at least one time difference selected from the group of:
- time difference between bolus injection and artery fluorescent signal or vein fluorescent signal,
- time difference between artery fluorescent signal and vein fluorescent signal,
- time difference between artery fluorescent signal or vein fluorescent signal and tissue fluorescent signal.

It may alternatively be formulated as continuously generating and/or receiving a fluorescent signal from (blood vessels in) the tissue wherein a phase difference in the fluorescent signal from blood vessels and surrounding tissue is repeatedly and/or continuously imposed.

As disclosed in PCT/EP2019/065648 one way of providing an oscillating fluorescent signals is to inject micro-boluses of ICG at regular intervals over a long period such that the intensity of the fluorescent signal will oscillate with a predetermined frequency, i.e. each time a bolus is injected, the fluorescent signal will appear subsequently thereafter and the frequency of the oscillation is determined by the time of bolus injection. As exemplified herein the micro-boluses makes it possible to continuously inject ICG at controllable, predetermined and/or regular intervals over a period of one hour or even several hours, e.g. regular intervals of between 1 and 10 minutes, e.g. 1-2 minutes, 2-3 minutes, 3-4 minutes, 4-5 minutes, 5-6 minutes, 6-8, minutes or 8-10 minute intervals.

Because the fluorescent signals from the small boluses keeps coming again and again the blood vessels can thereby be identified again and again in the fluorescence images of the tissue, i.e. based on said time difference(s) and the oscillating fluorescent signal. I.e. the disclosed method may consequently further include the step of continuously acquiring fluorescence images of the tissue, and preferably also the step of analysing the fluorescence images and the associated varying and/or oscillating intensity, wavelength and/or phase thereby continuously identifying blood vessels in the tissue.

There are various known ways of analyzing fluorescent signals and thereby for example visualizing blood vessels. The key aspect of the presently disclosed approach is about when to look for arteries, veins, tissue, etc. The repeated injections of small boluses ensures that a fluorescent signal is repeatedly present for analysis. And the determination and/or knowledge of subject specific hemodynamic time constants ensures that one knows when to look, or when to expect, a signal from arteries, veins, tissue and/or other.

One or more subject specific hemodynamic parameters might be known in advance, e.g. one or more hemodynamic time constants. For example known from an earlier procedure. Hence, the present disclosure also relates to relate method for identifying blood vessels in tissue of a subject, e.g. during a medical procedure, the method comprising the steps of
- continuously acquiring fluorescence images of the tissue wherein a fluorescent signal is oscillating with a predetermined pattern having a frequency, the pattern preferably generated from a series of boluses of at least one fluorescent imaging agent, and wherein the series of boluses is administered with a predefined and/or controlled duration between subsequent boluses determining the frequency of the pattern,
- analysing at least part or all of the fluorescence images, and
- identifying, preferably continuously and/or repeatedly, blood vessels in the fluorescence images of the tissue based on said subject specific hemodynamic parameter(s) and the fluorescent signal oscillating with the predetermined pattern.

A further aspect of the present disclosure relates to a system for identifying blood vessels in tissue during a medical procedure of a subject, wherein the system is configured for continuously generating a fluorescent signal from blood vessels in the tissue wherein the intensity of the fluorescent signal is oscillating with a predetermined pattern, continuously receiving fluorescence images of the tissue, and analysing the fluorescence images and the associated oscillating intensity thereby continuously identifying blood vessels in the tissue. The system may comprise a controllable injection pump for holding at least one first fluorescence imaging agent, the injection pump being configured for injecting a series of predefined boluses of said first fluorescence imaging agent into a vein of the subject, thereby generating the fluorescent signal which is oscillating with the predetermined pattern. The system may accordingly be configured for carrying out the methods disclosed herein.

Identified blood vessels can consequently be visualized and mapped to medical personnel, for example before and during tissue dissection. The main advantage is that the presently disclosed approach, i.e. automated and continuous blood vessel identification and detection, can be executed in the background, while the surgeon is working in his normal white light camera image. E.g. the micro-bolus procedure can be started at the initiation of surgery and run in the background, monitored by a computer system that both measures and receives the fluorescent signal, and controls the interval and dosing of the micro-bolus regimen. The surgeon can then, at any time during the surgery, shift to a computer generated "blood vessel view" which shows the blood vessels in the area of interest. Advantageously the continuously identified blood vessels can be superimposed in real time into the white light images such that the otherwise hidden blood vessels appear in the white light images in real time as augmented reality objects.

The presently disclosed approach can be of great use for surgeons during surgery, in any organ and for any indication, in particular where the structure and anatomy of blood vessels might be of interest. It can also be used in plastic surgery to evaluate vessel structure and anatomy in e.g. a skin flap which is to be transplanted, where it can provide information on where to dissect the skin flap. Similarly it can provide important information when a skin flap has been transplanted to a recipient, where it can provide information about the vessel structure and about the success of the vessel anastomosis.

The presently disclosed approach employed within a surgical procedure can be seen as surgical decision support. But the use of the presently disclosed approach is not limited to use during a surgical procedure. The presently disclosed approach can advantageously also be applied to provide information on blood vessel structure and anatomy before surgery or after surgery, or even for monitoring of wound healing and blood vessel anatomy in patients not undergoing surgery at all. In such cases the presently disclosed approach can be seen as a medical examination tool, much like a CT-scan.

The present disclosure further relates to a computer program (product) having instructions which, when executed by a computing device or computing system, cause the computing device or computing system to carry out the method of identifying blood vessels in tissue of a subject as disclosed herein.

### Brief description of figures

**Figs. 1A** shows an example of an intensity curve after a bolus of ICG has been provided to a subject and **Fig. 1B** shows the corresponding intensity curves where the hemodynamic parameters perfusion slope, slope start, slope end max intensity, washout slope, washout start and washout slope end have been calculated and are indicated in the graph.
**Fig. 2A** shows an oscillating time intensity fluorescent curve wherein the oscillations are disrupted due to the onset of ischemia in a human subject.
**Fig. 2B** shows a zoom-in of the time interval around t = 3800 s of the previous graph wherein the onset of ischemia occurs.
**Fig. 2C** shows idealized data with and without ischemic conditions.
**Fig. 2D** shows idealized data wherein only parts of the oscillating time intensity fluorescence curve may be detected.
**Fig. 3A** shows continuous measurements of a human subject injected with micro boluses.
**Fig. 3B** shows a zoom-in of the interval indicated in Fig. 3A.
**Fig. 4** shows measurements of a human, subjected to venous occlusion, wherein the blood flow is only partially restricted.
**Fig. 5A** shows a snapshot of an ICG analysis tool running on a humanoid subject (right forearm). The image is taken at a very early stage, where a micro-bolus of ICG has just been administered and is beginning to enter the arteries.
**Fig. 5B** shows a snapshot a few seconds later than fig. 5A where a plurality of arteries can be identified.
**Fig. 6A** shows a snapshot a few seconds later than fig. 5B where the fluorescence intensity from the specific micro-bolus ICG is reaching its peak value.
**Fig. 6B** shows a snapshot approx. one minute later than fig. 6A and illustrates the washout phase wherein veins can be identified.
**Fig. 7** shows an edge-filtered version of fig. 6B.
**Fig. 8** shows an image where arteries have been identified in the sequence of images shown in figs. 5A-6B.
**Fig. 9** shows an image where veins have been identified in the sequence of images shown in figs. 5A-6B.
**Fig. 10** shows an image where the arteries and veins shown in figs. 8-9 are visually enhanced in red (arteries) and blue (veins) and superimposed in the image such that they are easily distinguishable.

### Detailed description

One embodiment of the present disclosure relates to a computer implemented method for identifying blood vessels in tissue of a subject, e.g. during a medical procedure, the method comprising the steps of continuously receiving a fluorescent signal from blood vessels in the tissue wherein the intensity of the fluorescent signal is oscillating with a predetermined pattern, continuously acquiring fluorescence images of the tissue, and analysing the fluorescence images and the associated oscillating intensity thereby continuously identifying blood vessels in the tissue.

The predetermined pattern may be characterized by a predefined frequency with a period of between 30 second and 15, such as between 1 and 10 minutes, or between 1-2, 2-3, 3-4, 4-5, 5-6, 6-8, or 8-10 minutes over a time period of at least 10 minutes, or at least 15 minutes, or at least 30 minutes, or at least 1 hour, or at least 2 hours, and wherein the fluorescent signal is oscillating in intensity in accordance with this predetermined pattern. As also explained herein this predetermined pattern can originate from controlled injection of a series of small boluses of at least one fluorescent agent, such as ICG.

In the preferred embodiment the identified blood vessels are continuously displayed and visualized, e.g. on a screen. Furthermore, the identified blood vessels may be combined to identify one or more networks of interconnected blood vessels.

White light images of the tissue may also be continuously received and acquired. Hence, at least one white light image of the tissue may be generated wherein the identified blood vessels are visually enhanced, e.g. by superimposing the identified blood vessels into the white light images, preferably also visually enhancing the blood vessels, e.g. by means of high contrast colours, and displayed on a screen such that the identified blood vessels appear as augmented reality objects.

The preferred embodiment of the presently disclosed approach comprises the step of distinguishing arteries and veins in the identified blood vessels, this distinguishing between arteries and veins is advantageously based on the predetermined pattern of the oscillating fluorescent signal. The bolus hemodynamics is different for arteries and veins and when a bolus of e.g. ICG extends through a patient the fluorescent signal will initially appear in the arteries, then in microcirculation in the surrounding tissue and after a while in the veins. Hence, time differences between bolus injection, artery signal, tissue signal, and vein signal can for example be determined from the first or the first few boluses by analysing a series of corresponding images and look for signals in that order. I.e. the presently used novel micro-bolus dose regime imposes a multitude of time and/or wavelength dynamics that can be utilized. Because only one, or a few, micro-bolus of contrast agent is necessary to determine the patient specific / situation specific time difference between bolus injection and artery signal and between the artery signal and the vein signal, these time differences can be utilized in subsequent controlled micro-bolus administrations to continuously distinguish arteries and veins. The time differences between artery signal and tissue signal and between tissue signal and vein signal can also be utilized. Also the washout period of each micro-bolus involves different hemodynamics for arteries, veins and surrounding tissue which can be utilized to identify blood vessels and distinguish between arteries and veins, even during a washout period between bolus injections.

One example of identifying blood vessels in fluorescence images is by means of image filtering, preferably edge filtering, of the acquired fluorescent images. Due to the oscillating fluorescent signal and the hemodynamics in the tissue, there will almost constantly be regions in the acquired images where the image brightness changes sharply, i.e. it has discontinuities. These areas are most often either arteries or veins and hence by constantly applying appropriate image filtering, blood vessels will appear substantially constantly during the continuous bolus administration.

Another more accurate approach is to identify blood vessels based on an inherent phase difference between fluorescent signals associated with blood vessels and surrounding tissue, respectively. This is due to the hemodynamics in the body of the subject / patient. The overall oscillating fluorescent signal as disclosed herein is imposed by controlled and repeated injections of small boluses of a fluorescent agent like ICG. Each bolus of the fluorescent agent will also give rise to varying fluorescent signals on a more local level due to the hemodynamics in the subject. The fluorescent agent of each small bolus will reach arteries, tissue and veins at different time points and the presently disclosed approach utilizes these time differences to identify blood vessels, to distinguish blood vessels and tissue and to distinguish arteries, veins and tissue. The time differences between fluorescent signals originating from arteries, tissue and veins, respectively, can be seen as phase differences in a continuously evolving fluorescent signal. I.e. at any time during the oscillating fluorescent signal there are phase differences at different positions in the corresponding acquired fluorescent image resulting from the varying hemodynamics in arteries, veins and surrounding tissue, i.e. due to the inherent time differences originating from the hemodynamics of the subject. Either the subject specific time differences are known or the first one of few boluses can be used to determine the subject specific time differences, i.e. determining at least one time difference selected from the group of: time difference between bolus injection and artery fluorescent signal or vein fluorescent signal, time difference between artery fluorescent signal and vein fluorescent signal, and time difference between artery fluorescent signal or vein fluorescent signal and tissue fluorescent signal. One or more of these time differences can be "converted" to corresponding phases / phase differences or one or more of blood vessels, arteries, veins, surrounding tissue and/or something else. Hence, knowing the expected phases of arteries, veins and surrounding tissue it is thereby possible to associate each pixel, or ROI in the fluorescent image with a classification of either artery, vein, surrounding tissue or something else.

The expected phases of arteries, veins and surrounding tissue at a given point in time can be directly related to the predetermined oscillating pattern which determines the period and oscillation frequency of the oscillating fluorescent signal. The expected phases can either be approximated, calculated and/or determined during the medical procedure, e.g. as an initial learning period where the oscillating fluorescent signal is observed for one or a few micro-boluses to measure the associated hemodynamics in the specific situation. Thereby the expected phase of artery, vein and surrounding tissue can be directly related to the time point where a bolus is injected. Hence, in the subsequent medical procedure the time point of each bolus injection provides information of the expected phase of artery, vein and surrounding tissue.

Hence, the preferred embodiment of the presently disclosed approach comprises the step of determining a phase difference between a fluorescent signal originating from arteries in the tissue and a fluorescent signal originating from veins in the tissue and optionally a fluorescent signal originating from the tissue surrounding the blood vessels and relating this phase difference to the predetermined oscillating pattern.

Analysing phases and/or phase differences in a sequence of fluorescence images is therefore one way to identify blood vessels in the fluorescent images. I.e. blood vessels can be identified / detected in a fluorescent image sequence on a pixel by pixel level by knowing the phase of the fluorescent signal - and this can be detected anytime during the micro-bolus dose regime. This phase information coupled with knowledge of the predetermined bolus administration, i.e. that the fluorescent signal is oscillating with a predetermined pattern, provides the necessary information whether a pixel, or a region of interest, in an image is a blood vessel or not and even whether a pixel, or a region of interest, in an image is an artery, a vein, tissue or something else. Thereby actual mapping of the blood vessels, including arteries and veins, in substantially each fluorescent image can be provided, e.g. a pixel-by-pixel, or groups of pixels, or ROI-by-ROI, mapping in the form of each pixel - or ROI - being classified as blood vessel (preferably including artery or vein), surrounding tissue or optionally otherwise.

Taken all together the presently disclosed approach can provide medical personnel with a constantly updated map of the blood vessels, including arteries and veins, in the surgeon's field of view / anatomical region of interest. In particular the identified arteries and veins can be superimposed into white light images, visually enhanced such that arteries and veins are visually distinguishable, e.g. each having a separate high contrast colour, and displayed on a screen.

Once identified, and possibly mapped, in a fluorescent image, or sequences thereof, the blood vessels, preferably including identified arteries and veins, can be tracked in subsequent fluorescent images even in movement, such as peristaltic movement, is involved. Tracking of identified blood vessels can for example be provided by using tracking methods available in the prior art and known to the skilled person. Examples of tracking in fluorescent images are disclosed in WO 2018/104552.

In a further embodiment the presently disclosed method further includes the step of administering a series of boluses of at least one fluorescent imaging agent into (a vein) of the subject thereby generating the predetermined pattern of oscillating fluorescent intensity and/or wavelength. The series of boluses is preferably administered with a predefined duration between subsequent boluses.

The preferred fluorescent imaging agent used is ICG and each bolus of ICG preferably corresponds to less than 0.01 mg ICG/kg body weight of the subject, more preferably less than 0.005 mg ICG/kg body weight, even more preferably less than 0.004 mg ICG/kg body weight, yet more preferably less than 0.003 mg ICG/kg body weight, yet even more preferably less than 0.002 mg ICG/kg body weight, most preferably less than 0.001 mg ICG/kg body weight. The series of boluses may be injected automatically by a controllable, e.g. computer controlled, injection pump.

The imaged tissue may be part of an internal organ of the subject. The imaged tissue may further be part of an anatomical structure in the gastrointestinal tract, preferably selected from the buccal cavity; pharynx; the small intestine including duodenum, jejunum, and ileum; the stomach, including esophagus, cardia, and pylorus; the large intestine including cecum, colon, rectum and the anal canal. I.e. the imaged tissue may be subject to peristaltic movement during the medical procedure.

Alternatively the imaged tissue may be part of the skin of the subject. E.g. the imaged tissue is part of a wound of the subject.

### System

As previously described the present disclosure further relates to a system for identifying blood vessels in tissue during a medical procedure of a subject, the system configured for
- continuously generating a fluorescent signal from blood vessels in the tissue wherein the intensity of the fluorescent signal is oscillating with a predetermined pattern,
- continuously receiving fluorescence images of the tissue, and
- analysing the fluorescence images and the associated oscillating intensity thereby continuously identifying blood vessels in the tissue.

In the preferred embodiment the system further comprises a controllable injection pump for holding at least one first fluorescence imaging agent, the injection pump being configured for injecting a series of predefined boluses of said first fluorescence imaging agent into a vein of the subject, thereby generating the fluorescent signal which is oscillating with the predetermined pattern.

The fluorescence images may be received by an appropriate imaging unit, such as a camera, e.g. a surgical, laparoscopic or microscope camera, such as a video camera, which can be part of the presently disclosed system. The analysis may be provided by a processing unit, either locally or as part of a cloud service.

The presently disclosed system may be configured to carry out all the steps of the presently disclosed method.

The predefined bolus preferably corresponds to less than 0.01 mg ICG/kg body weight of the first fluorescence imaging agent. A predefined bolus may also correspond to less than 0.5 mg ICG of the first fluorescence imaging agent. Preferably he fluorescence agent is ICG and the amount of ICG in a predefined bolus is preferably less than 0.01 mg /kg body weight of the subject. Hence, preferably the amount of ICG in a predefined bolus is less than 1 mg ICG or less than 0.5 mg ICG.

The system may be configured to inject boluses with an interval between 5 and 600 seconds, such as between 15 and 300 seconds, for example between 45 and 210 seconds, such as between 90 and 120 seconds.

A further advantage of the presently disclosed approach is the opportunity to identify a local network of blood vessels. By clamping a freely visible blood vessel for a short period, the associated perfusion area is delimited and by subsequently observing the fluctuating ICG signal the associated network becomes clearly visible because the perfusion in that area rapidly changes.

### Intraoperative Fluorescence imaging

Perfusion (e.g. blood flow) can be imaged intra-operative and assessed in real time using the near-infrared light from a surgical microscope or camera and acquiring video of fluorescent light in the near-infrared region that is excited from a fluorescent vascular contrast agent that has been intravenously administered as a tracer. The state of perfusion during the operation can thereby be confirmed in real-time. In this disclosure the perfusion in blood vessels is used to identify blood vessels utilizing fluorescence imaging, but not necessarily limited to intra-operative use of a surgical camera.

The presently disclosed system and method can provide enhanced information of tissue characteristics including location of superficial and deeper blood vessels, in particular if different fluorescent agents are used, because careful selection of different fluorescent agents provides the option of having perfusion information from different depths in the tissue.

During a medical procedure, e.g. diagnostic, screening, examining and/or surgical procedure involving fluorescence imaging a solvent comprising the fluorescent contrast agent, such as ICG, is injected intravenously and the molecules are excited by an infrared light source, e.g. a laser with a wavelength in the infrared wavelength range, e.g. around 780 nm. Fluorescent light with a wavelength of around 830 nm is then emitted from the excited contrast agent molecules and can be recorded with an imaging device, e.g. in the form of a camera. A filter can be provided to block the excitation light as the excitation intensity typically is much larger than the fluorescence intensity. The excitation intensity can be around 1 W per emission angle whereas the fluorescent power pr. pixel can be around 0.15 pW. In spite of the several orders of magnitude in difference, good Signal to Noise Ratio (SNR) can be achieved. The recorded fluorescent light provides an image of the perfusion in imaged tissue and makes it possible to see deeper lying blood vessels, due to a penetration depth of 5-10 mm for ICG. Since the ICG molecule is bound to proteins in the blood, the video images contain information about the level of perfusion - but that information can be difficult to quantify for the surgeon during the operation if only the acquired video images are seen.

In the system and method of the present disclosure the fluorescent contrast agent is selected from the group of: indocyanine green (ICG) and fluorescein isothiocyanate, rhodamine, phycoerythrin, phycocyanin, allophycocyanin, ophthaldehyde, fluorescamine, rose Bengal, trypan blue, fluoro-gold, green fluorescence protein, a flavin, methylene blue, porphysomes, cyanine dye, IRDDye800CW, CLR 1502 combined with a targeting ligand, OTL38 combined with a targeting ligand, or a combination thereof.

**Indocyanine green (ICG)** is a cyanine dye used in medical diagnostics, it is far the most common dye used for perfusion assessment. It has a peak spectral absorption at about 800 nm. These infrared frequencies penetrate retinal layers, allowing ICG angiography to image deeper patterns of circulation than fluorescein angiography. ICG binds tightly to plasma proteins and becomes confined to the vascular system. It is administered intravenously and, depending on liver performance, is eliminated from the body with a half life of about 3-4 minutes by the liver to bile juice. ICG sodium salt is normally available in powder form and can be dissolved in various solvents; 5% (<5% depending on batch) sodium iodide is usually added to ensure better solubility. The sterile lyophilisate of a water-ICG solution is approved in many European countries and the United States under the names ICG-Pulsion, IC-Green and VERDYE as a diagnostic for intravenous use.

The absorption and fluorescence spectrum of ICG is in the near infrared region. Typically a laser with a wavelength of around 780 nm is used for excitation. At this wavelength, it is possible to detect the fluorescence of ICG by filtering out scattered light from the excitation beam.

The toxicity is ICG is classified as low but administration is not without risks, e.g. during pregnancy. It is known that ICG decomposes into toxic waste materials under the influence of UV light, creating a number of still unknown substances. I.e. it is within the interest of the patient that the dose of ICG used during fluorescence imaging is minimized, as demonstrated herein.

**Fluorescein** is another dye which is widely used as a fluorescent tracer for many applications. Fluorescein has an absorption maximum at 494 nm and emission maximum of 512 nm (in water). Hence, it would be suitable for use in combination with ICG because the absorption and emission wavelength of the two dyes are separated by several hundred nanometres.

### Dose regime

The present disclosure further relates to a method for automatic perfusion assessment of an anatomical structure of a subject, the method comprising administration into a vein of a bolus of about 1/10 of the normal dose used for perfusion assessment. For Indocyanine green (ICG), the normal bolus is 0.1.-03 mg/kg body weight. According to the present disclosure a bolus of less than 0.01 mg/kg body weight, preferably less than 0.005 mg/kg body weight, more preferably less than 0.0049 mg/kg body weight of a first fluorescence imaging agent like ICG can be used, even more preferably less than 0.0048 mg/kg body weight, yet more preferably less than 0.0047 mg/kg body weight, most preferably less than 0.0046 mg/kg body weight, and even more preferably less than 0.004 mg/kg body weight of a first fluorescence imaging agent can be used. For other fluorescent imaging agents described herein, the bolus is similarly reduced according to the present disclosure. As stated above the agent may be injected by a controllable injection pump, for example as a series of boluses with a predefined time between subsequent boluses. Following injection of each bolus the fluorescence emission from the anatomical structure can be measured. This method for automatic perfusion assessment of an anatomical structure of a subject can also be combined with the presently disclosed method for continuously identifying blood vessels in tissue, in particular with regard to the bolus administration regime.

The minimum bolus that provides a quantifiable fluorescence emission representative of the perfusion of the anatomical structure and/or identifiable blood vessels can be determined following administering a series of increasing boluses. The bolus may comprise incrementally increasing or incrementally decreasing amounts of the agent, for example the amount may increase or decrease in increments of 10% from one bolus to the subsequent bolus.

The boluses are preferably provided as a regular series of injections with a predefined and regular time interval each bolus. The interval between boluses can be between 5 and 600 seconds, such as between 5 and 300 seconds, such as between 10 and 180 seconds, such as between 10 and 140 seconds, such as between 10 and 90 seconds, such as between 15 and 80 seconds, for example between 20 and 70 seconds, such as between 30 and 60 seconds. In another embodiment the interval between boluses can be between 5 and 600 seconds, such as between 10 and 600 seconds, such as between 15 and 600 seconds, such as between 15 and 300 seconds, such as between 30 and 240 seconds, such as between 45 and 240 seconds, for example between 90 and 240 seconds, such as between 90 and 120 seconds. Preferably between 60 and 600 seconds, or between 120 and 600 seconds. The interval between boluses is preferably sufficiently long to allow measurement of a perfusion slope for each bolus in the anatomical structure, preferably wherein the perfusion slope includes a slope start and a washout slope.

For ICG the amount of fluorescence imaging agent is preferably between 0.0001 and 0.001 mg/kg body weight per bolus, such as between 0.001 and 0.01 mg/kg body weight per bolus, preferably between 0.0005 and 0.005 mg/kg body weight per bolus, more preferably between 0.001 and 0.004 mg/kg body weight per bolus. An initial amount of fluorescence imaging agent is advantageously at least 0.001 mg/kg body weight, and preferably less than 0.005 mg/kg body weight. Subsequent boluses may then increase by at least 0.001 mg/kg body weight and preferably less than 0.005 mg/kg body weight per bolus from one bolus to the subsequent. For other types of fluorescence imaging agents the dose is preferably chosen based on its fluorescence relative to ICG. Therefore a fluorescence imaging agent having higher emission rates is preferably administered at a correspondingly lower dose. The dose may for example be substantially inverse linear to the quantum yield of the fluorescence imaging agent. The dose may further be based on the absorption and emission spectra relative to ICG.

The bolus is preferably a liquid volume of between 0.5 µL and 10 mL, such as from 0.5-5 mL. I.e. the amount of the first fluorescence imaging agent is preferably dissolved in a liquid. In a preferred embodiment of the present disclosure a volume of isotonic solution (such as saline) is injected immediately following injection of a bolus of fluorescence imaging agent, for example wherein the volume of isotonic solution is 1-20 mL, such as 2.5-15 mL, for example 5-10 mL.

In a further embodiment of the present disclosure a second fluorescence imaging agent is administered, the second fluorescence imaging agent having an emission maximum differing from the emission maximum of the first fluorescence imaging agent by at least 50 nm, or by at least 100 nm. The first and second fluorescence imaging agents are preferably administered alternatingly. Advantageously the interval between administrations of different fluorescence imaging agents is half of the interval between subsequent administrations of the same fluorescence imaging agent.

In a further embodiment of the presently disclosed method a series of fluorescence images of the anatomical structure and/or tissue is formed for the assessment of perfusion and/or for identification of blood vessels. The fluorescence may be detected automatically by illuminating the anatomical structure / tissue with a light source capable of exciting the fluorescence imaging agent, and the emission is quantified and/or analysed through a series of fluorescence images of the anatomical structure / tissue.

The period between boluses can be determined by a computer configured to detect the perfusion slope caused by each bolus. Further, the amount of fluorescence imaging agent in a bolus may be controlled by a computer configured to determine a minimum bolus corresponding to a minimum fluorescence emission representative of the perfusion of the anatomical structure. This computer may be part of the presently disclosed system.

In a further embodiment the perfusion assessment comprises localizing a perfusion complication in the anatomical structure. Hence, the perfusion assessment may be used in connection with a diagnostic or surgical procedure, for example the procedure comprises diagnostic laparoscopy, explorative laparoscopy, surgical laparoscopy with traditional laparoscopy, robotic surgery, and open surgery. The procedure may alternatively comprise creation of anastomosis, such as intestinal anastomosis, wounds, plastic surgery, cardiac surgery or cancer.

A further embodiment of the present disclosure relates to a fluorescence imaging agent for use in the methods disclosed herein. Yet a further embodiment relates to the use of a fluorescence imaging agent in the preparation of a medicament for use in a method of automatic perfusion assessment and/or continuous identification of blood vessels as disclosed herein.

In a further embodiment of the present disclosure the fluorescence imaging agent is repeatably injected. In certain cases, there may be a need for a longer phase, such as at least 2 minutes, preferably at least 3 minutes, even more preferred at least 4 minutes, yet even more preferred at least 5 minutes, almost most preferably at least 8 minutes, most preferably at least 10 minutes, wherein the fluorescence imaging agent is not injected, in order to allow for the fluorescence imaging agent to be washed out, such that the background level is reduced. Once the background level is reduced to an acceptable level, such as below a certain percentage of the maximum fluorescent intensity or until substantially no fluorescent can be measured, the injections of the fluorescence imaging agent may be continued.

The presently disclosed system may be configured to carry out the presently disclosed methods. This may be provided by the system having at least one processor and memory having instructions stored thereon, instructions, when executed by the one or more processors, cause the system to carry out the presently disclosed method of automatic perfusion assessment of an anatomical structure.

### Oscillating dynamics

The inventors have further realized that the measurement and analysis of repeatable bolus injections can additionally be expanded from interpretation and quantification of a single inflow and/or a single outflow phase to analysis of oscillating fluorescence dynamics. These oscillating fluorescence dynamics may disclose physical perfusion characteristics hitherto unattainable without invasive measures.

The presently disclosed system and method may be configured for repeated injections of small boluses, such as the minimum bolus, at regular intervals. These boluses may, depending on for example the injection time interval, lead to a cyclic variation that when measured takes the approximate form of a sinusoidal curve. In such a curve, the measured intensity signal is expected to increase with the inflow of the fluorescence imaging agent from a given bolus, and thereafter decrease during the wash-out phase of the bolus, until it once again increases at the subsequent bolus and so forth, resulting in a cyclic (sinusoidal) pattern.

Preferably the presently disclosed system can be configured such that it can recognize parameters of the oscillating intensity curve, such as the frequency and/or amplitude. The trained system can then in turn anticipate both the direction and regularity of the forthcoming signal dynamics. The system preferably uses measured values in order to recognize the oscillating pattern, such that the system thereafter is able to detect discrepancies between measured values and expected values. The measured values may further be continuously used for improving the pattern recognition, i.e. the expected values. Alternatively or additionally, injection parameters such as the bolus frequency, dose and flow rate may be used for determining the expected values, i.e. the oscillating pattern.

With the system anticipating the expected value, it is able to, at an early time point - ideally instantaneously, detect and alert the onset of ischemic conditions. The detection of ischemic conditions may be a function of the expected value(s) and the detected value(s), such as for example a threshold value. However, with the presently disclosed approach of continuously identifying blood vessels, an ischemic condition, or any type of disruption or breach in a blood vessel or a network of blood vessels, can be observed visually almost instantaneously by the relevant medical personnel.

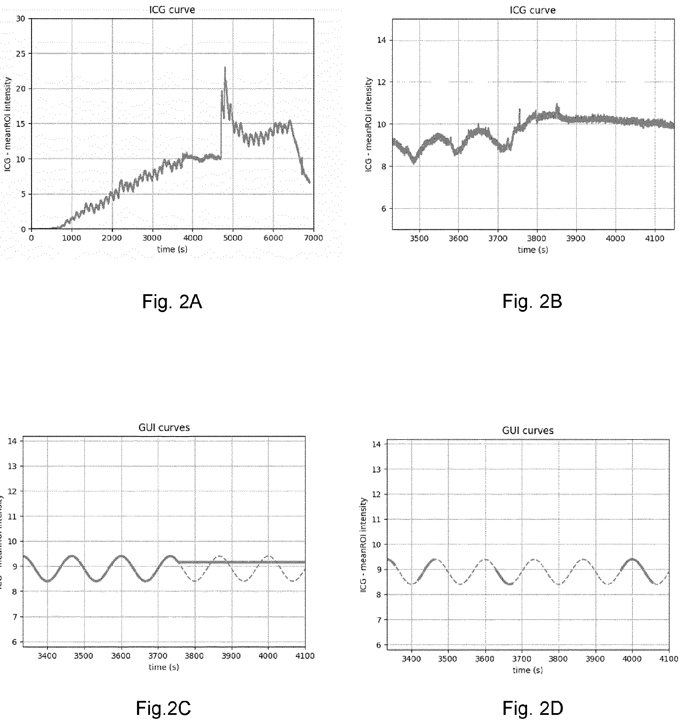

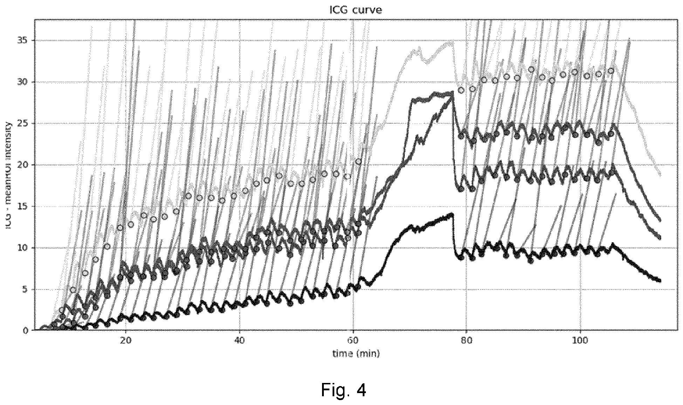

Discrepancies from the expected sinusoidal pattern may be caused by for example the onset of ischemic conditions in at least a part of an anatomical structure / tissue visible in the video image, or by a regional change in perfusion to a given area. An explanatory figure, demonstrating this change in dynamics due to the onset of ischemia in a human subject, is given in figure 2A and a more narrow zoom is given in figure 2B. As seen, it is possible to detect the transition from the regular oscillatory fluorescent signal to the ischemic flatline. It should however be noted that a change to the perfusion of the anatomical structure of interest may result in other measured patterns, additional to an ischemic flatline. An example is venous occlusion, wherein the outflow of blood from an anatomical area is blocked or reduced leading to a change in the oscillating dynamics due to congestion or pooling of fluorescent agents in the given area. As can be seen in Fig. 4, while the cyclic oscillations cease the result is not a flatline.

Such a system, as described herein, can observe and detect changes in the perfusion level of a given area in the video image within seconds. This can be detected in an area which has been observed for a prolonged time, such as many minutes, where the dynamics have been visualized continuously and the phase thus is well known. An explanatory figure highlighting the difference between signals one can expect to observe for ischemic/healthy tissue areas is shown in figure 2C. However, it can equally be determined in an anatomical area which has only been visualized during a short time interval, e.g. 10-20 seconds, as the described system is trained to expect and detect a certain phase of the described oscillating dynamic signal at a given time in the tissue, consisting of regular rises and falls in the time-intensity signal. Cf. figure 2D illustrating how it may look if the anatomic region of interest drifted in and out focus of the recorded image.

Preferably, the system comprises tracking means and is able to run independently in the background, while a surgeon is only exposed to the visible white light signal, and thus only interrupted/notified by warning signals. During for example the detection of the onset of ischemic conditions.

Another aspect of the present disclosure relates to continuous perfusion assessment in relation to repeated injections of a fluorescence active agent and monitoring the resulting oscillating curve. In addition to detect unforeseen changes to the perfusion, the system may be used for assessing the perfusion area of an artery. As an example a surgeon may consider to cut an artery as part of a surgical procedure. Before cutting the artery the surgeon may temporary restrict the perfusion through said artery and the presently disclosed approach can enable the visualization of the perfusion area of said artery within a short period of time, for example less than 1 minute. This may be valuable information for the surgeon during the continued surgical procedure. In a similar fashion, the system may be used for assessing the drainage area of a vein or a group of veins, lymph vessels, lymph nodes or other parts of the circulatory and/or lymphatic pathways. By temporarily restricting the blood flow through the vessel, the blood will pool up in the anatomical region which is normally drained by this vessel or group of vessels. This enables the visualization of the anatomical area which is drained by the vessel within a relatively short period of time, for example less than 2 minutes. This may provide important information to the surgeon, such as during the continued surgical procedure, in areas such as general surgery and plastic surgery, including wound and reconstructive surgery.

### Gastrointestinal tract

Complications related to the gastrointestinal tract are often related to local hemodynamics. I.e. a change in the normal hemodynamic conditions may be an indicator of increased risk of a complication. Perfusion assessment of the gastrointestinal tract, in particular in and near the surface of the gastrointestinal tract, such as the tissue of the gastrointestinal wall, can therefore be an important diagnostic tool when examining the gastrointestinal tract, e.g. for diagnosis or for localization of a complication, for example during diagnostic laparoscopy, explorative laparoscopy or surgical laparoscopy with traditional laparoscopy or robotic surgery, as well as in open surgery. Perfusion assessment is also important during the surgical procedure of creating an anastomosis which can be provided to establish communication between two formerly distant portions of the gastrointestinal tract. As an example intestinal anastomosis establishes communication between two formerly distant portions of the intestine and typically restores intestinal continuity after removal of a pathologic condition affecting the bowel. Intestinal anastomosis may for example be provided for 1) restoration of intestinal, such as bowel, continuity following resection of diseased intestine, and 2) bypass of unresectable diseased intestine, e.g. bowel. Certain paediatric conditions may also require intestinal anastomosis [6].

Resection of diseased bowel can be performed in the following settings:
- Bowel gangrene due to vascular compromise caused by mesenteric vascular disease, prolonged intestinal obstruction, intussusceptions, or volvulus
- Malignancy
- Benign conditions (e.g. intestinal polyps, intussusception, roundworm infestation with intestinal obstruction)
- Infections (e.g. tuberculosis complicated with stricture or perforation)
- Traumatic perforations
- Large perforations (traumatic) not amenable to primary closure
- Radiation enteritis complicated with bleeding, stricture, or perforation
- Inflammatory bowel disease, ulcerative colitis, or Crohn's disease that is refractory to medical therapy or associated with complications (e.g. bleeding, perforation, toxic megacolon, dysplasia/carcinoma)
- Chronic constipation, idiopathic slow transit constipation, or Hirschsprung's disease: Subtotal colectomy may be performed when the disease is refractory to medical therapy.

Bypass of unresectable diseased bowel can be performed in the following settings:
- Locally advanced tumour causing luminal obstruction
- Metastatic disease causing intestinal obstruction
- Poor general condition or condition that prevents major resection

Paediatric conditions for which intestinal anastomosis may be required include the following:
- Congenital anomalies (e.g. Meckel diverticulum, intestinal atresia, malrotation with volvulus leading to gangrene, meconium ileus, duplication cysts, Hirschsprung's disease)
- Inflammatory conditions (e.g. necrotizing enteritis, enterocolitis, tuberculosis, enteric perforation)
- Other conditions (e.g. intussusception, angiodysplasia, polypoid disease, ascariasis)
- As a part of other surgical procedures (e.g. Kasai portoenterostomy, choledochal cyst, urinary diversions, pancreatic neoplasms)

Postsurgical complications in connection with anastomosis in the gastrointestinal tract are unfortunately frequent, often due to insufficient perfusion (capillary blood supply) at the anastomosis, i.e. the joining of the two parts of the tract. Insufficient perfusion may cause anastomotic leakage, which is a serious and frequent complication, for example in connection with colorectal surgery where more than 10% of the procedures result in complications. Within colon cancer surgery more than 30% of patients with anastomotic leakage die due to postoperative complications and approx. 25% of the remaining patients suffer from stoma for the rest of their lives. Risk factors associated with leakage include tension of anastomosis, tissue damage and in particular reduced blood perfusion.

The present disclosure therefore in one embodiment relates to performing image analysis of one or more video sequences representing at least a part of the gastrointestinal tract, for example acquired before, during and/or after surgery, in particular surgery involving the gastrointestinal tract. This may in particular apply to gastrointestinal surgery - the video sequence may therefore comprise at an exterior portion of at least a part of the gastrointestinal tract, preferably such that perfusion in at least a part of the gastrointestinal wall can be measured and assessed.

The gastrointestinal tract is an organ system within humans and other animals which takes in food, digests it to extract and absorb energy and nutrients, and expels the remaining waste as faeces and urine. The gastrointestinal tract can be seen as a tube that transfers food to the organs of digestion. The term gastrointestinal tract as used herein therefore includes the buccal cavity; pharynx; the small intestine including duodenum, jejunum, and ileum; the stomach, including esophagus, cardia, and pylorus; the large intestine including cecum, colon, rectum and the anal canal.

### Clinical applications

Visualization of blood vessel anatomy as disclosed herein is of great importance during almost any kind of surgery as the continuous detection of blood vessels will reduce the risk of unintended cutting a blood vessel. The presently disclosed system and method can in particular also be employed within the following clinical applications:

### ABDOMINAL/GENERAL SURGERY

- **Resection surgery,** to quickly locate correct blood vessels to be cut/ligated
- **Ischemic bowel surgery,** to quickly determine which and where blood vessels are blocked and anatomy insufficiently perfused.
- **Acute abdomen,** to help discover the underlying pathology, e.g. rule out ischemia.
- **Repeated surgery,** surgery in previously operated patients having a lot of surgical adhesions.
- **Cancer surgery performed by general surgeons,** in order to detect blood vessel, like in resection surgery.
- **General surgery,** involving substantially any organ in the abdominal tract. e.g. when performing anastomosis or surgery on the stomach (ventricle).
- **General surgery,** involving infections, superficial or deep, to detect and map blood vessels.

### THYROID SURGERY

- **Thyroid surgery,** including resection of thyroid tissue. Continuous detection of blood vessels is a major advantage as thyroid surgery carries a risk of excessive bleeding. Thyroid surgery also carries a risk of dissecting or removing part of one or more of the parathyroid glands and the presently disclosed approach can be applied for identifying and mapping blood vessels in the relevant area such that the parathyroid glands are more visible to the surgeon.

### PELVIC SURGERY

- **Gynecologic/urogynecologic surgery,** in order to quickly locate correct blood vessels to be cut/ligated, and to identify and distinguish blood vessels during surgery.
- **Cancer surgery,** in order to detect blood vessel.

### PLASTIC SURGERY

- **Skin transplantation,** in order to quickly locate correct blood vessels in donor to be cut/ligated, and to monitor the perfusion of the same blood vessels on the recipient; both in the acute setting during surgery, but also in the days that follow, in order to monitor healing and blood vessel formation.
- **In all skin near surgical procedures,** in order to detect and map the blood vessel structure in an anatomical region before the surgical procedure is initiated, and during the surgical procedure if the surgeon deems this necessary for additional information. I.e. the presently disclosed approach can be used as a clinical tool to identify map a patient's blood vessels and the associated blood vessel anatomy days or weeks before a surgical procedure is to be carried out, giving the medical personnel time to plan the procedure carefully. This mapping can be carried out according to the presently disclosed approach, that measures the fluorescent signal through the skin in the area of interest for e.g. 30-45 minutes, and creates a 2D or 3D map for the medical personnel to use in the planning. A map of identified blood vessels can be integrated with other examinations, such as CT-, MR- or ultrasound scans. The presently disclosed approach has the advantage that it allows for identification and mapping of blood vessels which are too small to be mapped precisely in e.g. a CT-scan.

### EAT THROAT AND NECK SURGERY

A variety of procedures including: **Facial cosmetic surgery, tracheostomy, cancers, etc.**

### ORTHOPEDIC SURGERY

- **Amputations,** in order to quickly identify and map blood vessels before a limp or anatomical region is amputated, to choose the correct site of amputation and the best site to close the skin again to secure optimal healing.
- **Infections etc.,** in order to detect and map blood vessels where debridement or similar procedures are necessary.

### CARDIAC SURGERY

**CABG,** in order to quickly identify and locate marginal blood vessels to the heart when performing bypass surgery.

### VASCULAR SURGERY

**Amputation surgery,** in order to quickly identify and locate correct blood vessels to be cut/ligated as above,

**Harvesting of blood vessels for bypass surgery,** in order to quickly identify and locate correct blood vessels to be cut/ligated.

### Examples

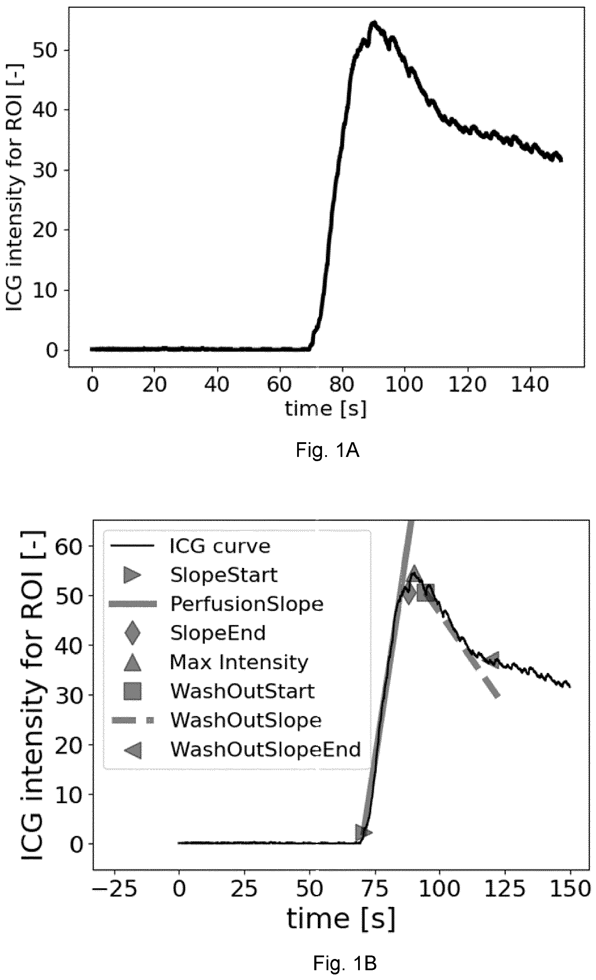

The intensity curves shown in figs. 1A-B are results of injections of boluses with normal amounts of fluorescent agent, in these cases ICG. In the amount of ICG in each bolus was chosen such that fluorescence emission was visible to the human eye. The examples are provided to illustrate the various perfusion parameters that can be calculated following fluorescent imaging. These same parameters can to a large extent also be determined following injection of the much smaller doses, i.e. the micro-dose approach with possibly repeated and continuous measurements and related assessment of perfusion and identification of blood vessels, which is disclosed herein.

Fig. 1A show an example of an intensity curve acquired from tissue after a bolus of ICG has been provided to a subject, e.g. from a region of interest in a video sequence. The same kind of data could be obtained if another contrast agent was used. The intensity is substantially zero until a steep rise in intensity indicates the passage of ICG molecules in the imaged tissue, the ICG molecules being excited to fluoresce. The peak in intensity is followed by the gradual washout of the ICG molecules. The intensity is indicated with arbitrary units.

Fig. 1B shows the corresponding intensity curve where the hemodynamic parameters perfusion slope, slope start, slope end max intensity, washout slope, washout start and washout slope end have been calculated and are indicated in the graphs. Assessment of perfusion parameters are further disclosed in pending application WO 2018/104552.

Fig. 2A shows actual measurement data from a human subject. The human subject is repeatably injected with micro boluses of ICG at regular intervals (in this example with intervals of around 2 minutes). The initial micro bolus of ICG comprised an amount of 0.00456 mg ICG/kg body weight of the human subject and each of the subsequent micro boluses of ICG comprised the same amount of 0.00456 mg ICG/kg body weight of the human subject. The time intensity curve shows a substantially sinusoidal pattern which increases linearly over time. The increase in intensity over time is related to the proportion between the dose of the fluorescent agent and the wash out time, during which the fluorescent intensity decreases. At a certain time point, around t = 3800 s, Fig. 2B, the perfusion is restricted causing an onset of ischemia, which can be seen by the lack of oscillations following this time point, forming what can be described as an ischemic flatline.

Fig. 2C shows idealized data displaying a sinusoidal time-intensity curve. The measured ROI intensity increases upon injection of a fluorescence imaging agent and decreases during the wash-out phase. At approximately t = 3750 s the measured data shows a stationary measured ROI intensity value due to the onset of ischemic conditions. Alternatively, had there not been ischemic conditions, the measured values are instead expected to follow the dashed line, such that the measured ROI values continuously follows the sinusoidal pattern.

Fig. 2D shows idealized data displaying a sinusoidal time-intensity curve without ischemic conditions wherein the anatomical region of interest drifts in and out of focus. The dashed line shows the expected measurement values if the ROI would be continuously observable. If this is not possible, for example due to the anatomical region of interest drifting in an out of focus of the recorded image, the measured data may not be complete but instead gaps - time intervals wherein no measurement data of the anatomical region of interest have been acquired - may be present. Therefore, the system is preferably able to recognize the sinusoidal pattern even when the recorded data is not complete because the phase of dynamics is known. If the system is able to correctly recognize the sinusoidal pattern, it is provided with expected intensity values of the ROI at each time point, which thereafter can be used for comparison with measured values. If the measured value(s) differs from the anticipated value(s) the system may be configured to provide the surgeon with an alarm. Therefore, the system may be configured such that it recognizes the phase of the oscillating/sinusoidal pattern of a measured time point or interval, which is thereafter compared to an expected phase of the time point or interval, wherein the expected phase is preferably based on the recognized oscillating pattern and/or the known frequency of the repeated bolus injections. As a result, the system does not necessarily require continuous measurement values, but may instead be based on the expected phase of the oscillating pattern in combination with the time information of the measured time point or interval, such that a specific phase of the oscillating pattern is expected to be present in the measured interval.

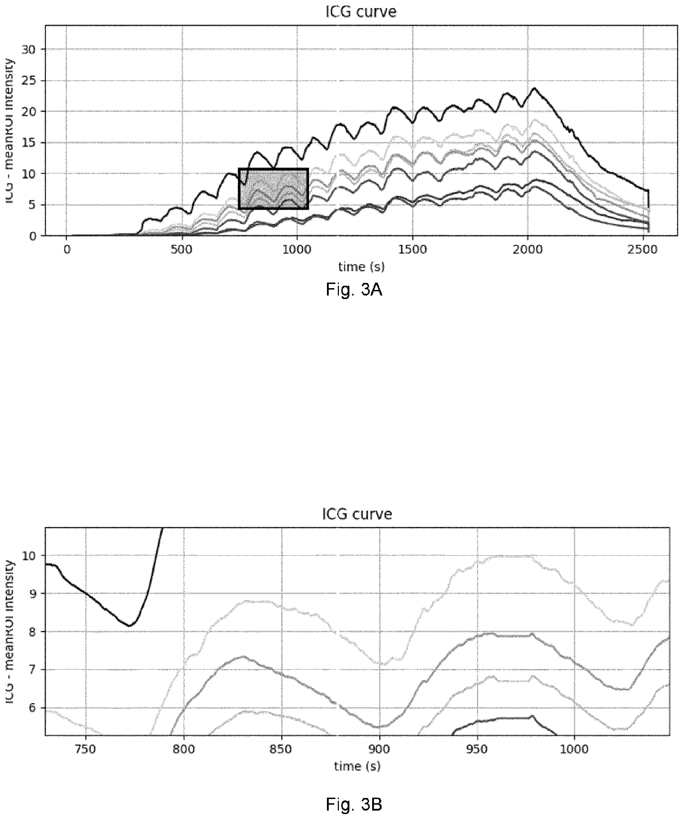

Fig. 3A shows a fluorescent intensity measurement of a human subject conducted over a longer time interval, approximately 40 minutes, wherein the human subject has been repeatedly injected with micro boluses of ICG. The initial micro bolus of ICG comprised an amount of 0.006 mg ICG/kg body weight of the human subject and each of the subsequent micro boluses of ICG comprised the same amount of 0.006 mg ICG/kg body weight of the human subject. The intensities of seven separate ROIs where measured, and have been assigned separate colours in the graph. The measured fluorescence intensities show cyclic sinusoidal patterns wherein the frequencies agree with the injection frequency (around 120 s). The patterns are substantially linearly increasing due to the accumulation of the fluorescence imaging agent, due to the relatively short period of the injections in comparison to the dose size. At approximately *t =* 2000 s, the repeated injections of the fluorescence imaging agent are stopped causing an approximately exponential decay of the fluorescence intensity.

Fig. 3B shows a zoom-in of the marked region in Fig. 3A. Here, the smaller fluctuations within the same ROI as well as between different ROIs can be seen. At the same time, the cyclic intensity patterns are distinct with the pattern of each ROI have an identical period.

Fig. 4 shows time-intensity plots of a measurement carried out on a human subject by repeatable injections of micro boluses of the fluorescence imaging agent. The initial micro bolus of ICG comprised an amount of 0.00456 mg ICG/kg body weight of the human subject and each of the subsequent micro boluses of ICG comprised the same amount of 0.00456 mg ICG/kg body weight of the human subject. The graph shows the result of a venous occlusion wherein, between approximately t = 62-78 minutes, the perfusion is restricted, while not completely hindered. In this case, the oscillating dynamics of the measured fluorescence intensities ceases and the measurements display an irregular increase during the venous occlusion. Therefore, it should be noted that decreased perfusion does not necessarily result in a flatline, as is otherwise typically the result during ischemic conditions.

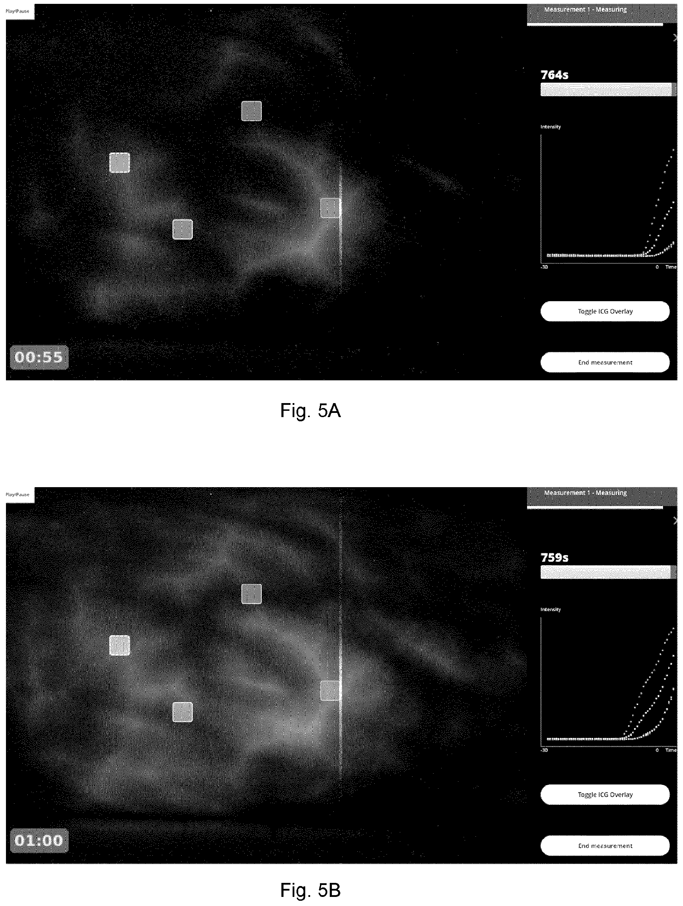

Fig. 5A shows a snapshot of an ICG analysis tool running on a humanoid subject. The images acquired in figs. 5-10 shows a section of the right forearm, i.e. the fluorescence signal is seen through the skin on the arm. The image in fig. 5A is taken at a very early stage of a bolus injection, where a micro-bolus of ICG has just been administered and is beginning to enter the arteries, i.e. some arteries can be identified. Four boxes in the image indicate measuring regions, aka regions of interest (ROI), and four ICG intensity curves are shown on the right, one for each ROI. One of the ROIs is located on and artery and the corresponding intensity curve is the highest. One ROI is located on a tissue area and the associated intensity curve shows that some ICG has already diffused in to the tissue. Two ROIs are located on veins and are almost flat indicating that ICG is yet to exit the tissue area to be transported back through the veins. The intensity curves of the two vein ROIs are coincident and cannot be distinguished from each other. The phase differences of the four ICG intensity curves are clearly visible. Hence, if the expected phase of arteries, veins and tissue were known, the four ROIs in fig. 5A could be classified as artery, vein, and surrounding tissue, respectively.

Fig. 5B shows a snapshot a few seconds later than fig. 5A where a plurality of arteries can be identified visually. Still there are several dark areas where the ICG has yet to spread, i.e. ICG is still entering the subject. All four ICG intensity curves of the four ROIs are steadily increasing. However, as seen from the curves there are distinct phase differences for the three groups of curves: 1) arteries, 2) tissue, and 3) veins). The artery ROI is ahead of the two other groups. The tissue ROI is 'behind' the artery curve. And the tissue ROI is "ahead" of the vein ROIs. Notice, that the phase differences in both directions are more or less equal, i.e. the tissue is approx. midway on the ICG molecules journey from arteries to tissue to veins. Again the ROIs can be classified as either artery, vein, surrounding tissue or other if the expected phase is known. This can be provided for all pixels in the image, or groups of pixels, i.e. all pixels can be classified as artery, vein, surrounding tissue, or other, if the expected phase is known. I.e. image filtering can provide a visual identification of blood vessels, but if the phase of the various signals is known relative to the predetermined oscillating pattern, all or most of the pixels in the images can be classified, i.e. much more detailed information is obtained. This can for example be utilized when superimposing the blood vessels into white light images and also when it is needed to track objects in a sequence of images where movement, e.g. peristaltic movement, takes place.

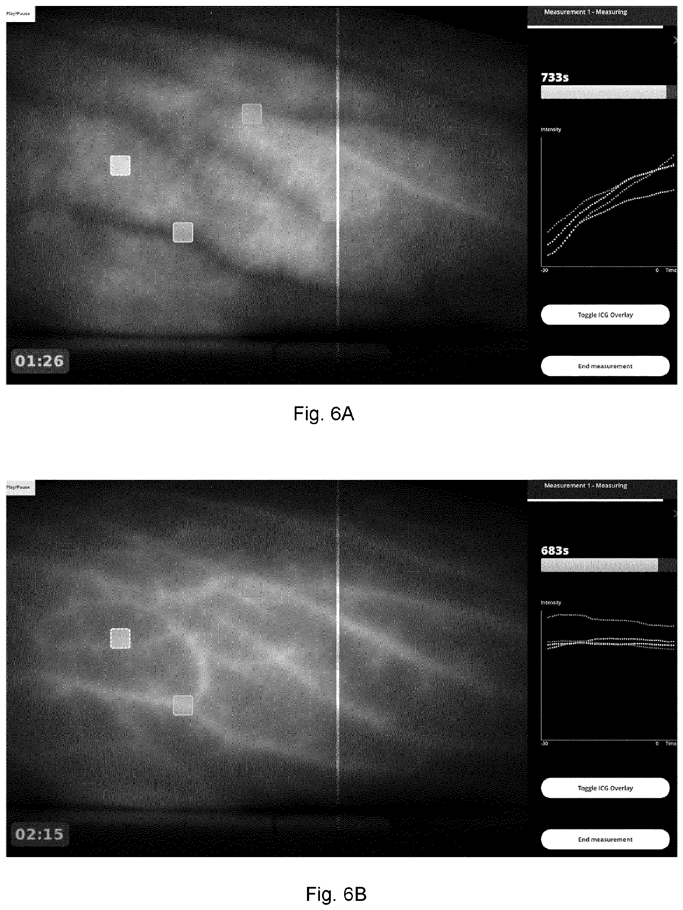

Fig. 6A shows a snapshot a few seconds later than fig. 5B where the fluorescence intensity from the specific micro-bolus ICG is reaching its peak value. After this point, more ICG will start to leave the tissue area than the amount of ICG which enters it. Comparing the image to earlier shown snapshots in figs. 5A-B it is seen that almost all areas are now visible. The darkest areas are now the veins, which have yet to start transporting the ICG away from the tissue. This is also reflected in the corresponding ICG intensity curves to the right. The ICG curves are all still increasing, i.e. we are still in the artery-dominated phase, but the concave shape of the curves indicated the peak intensity is approaching. But a phase difference is still seen between the ICG intensity curves. In this snapshot the blood vessels are not clearly identifiable because too much ICG has entered the tissue area from the arteries and not enough ICG has entered the veins yet. However, the veins can actually be identified as dark areas.

Fig. 6B shows a snapshot approx. one minute later than fig. 6A and illustrates the washout phase wherein veins can be clearly identified. The corresponding perfusion analysis from the associated ICG curves shows that the vein ROIs clearly have the highest intensity but also that the time scale is different in the washout phase, i.e. it may require a longer timescale to assess the phase differences between arteries, tissue and veins in the washout phase. But phase differences are visible in the ICG curves.

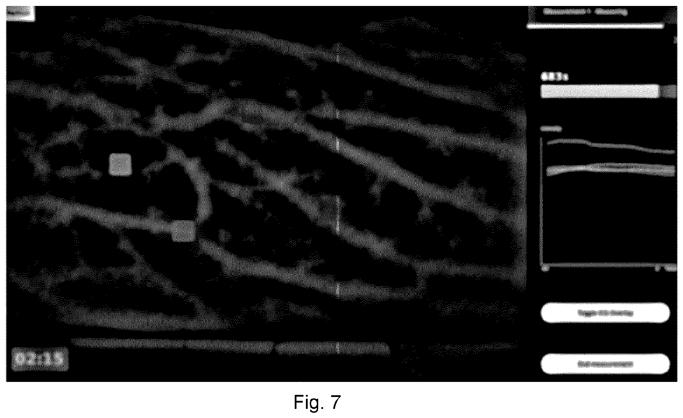

Fig. 7 shows an edge-filtered version of fig. 6B illustrating an example of a visual enhancements which can be provided for medical personnel during a medical procedure by exploiting the presently disclosed approach. Once the phase differences are known for the specific situation it is known when to optimally identify arteries and veins, respectively. In fig. 7 the veins are visually enhanced which would allow the surgeon to avoid accidentally cutting any veins. A pixel-by-pixel approach is used in fig. 7 in combination with edge-filtering to fig. 6B followed by a smoothing filter. The result clearly darkens the entire image except for the main veins. This information could be overlayed / superimposed on any screen, also a white light image screen, visual to the surgeon during surgery to improve the foundation on which the surgeons draws his decisions.

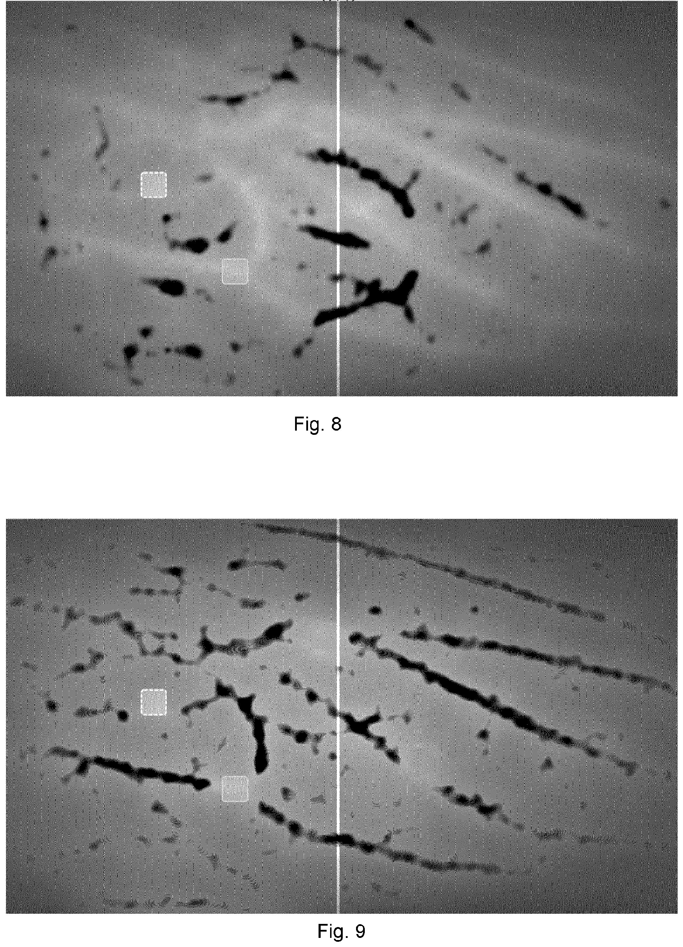

Fig. 8 shows an image where arteries have been identified in the sequence of images shown in figs. 5A-6B. The arteries are visually enhanced in black such that they are visible in a grey-scale image.

Fig. 9 shows an image where veins have been identified in the sequence of images shown in figs. 5A-6B. The veins are visually enhanced in black such that they are visible in a grey-scale image.

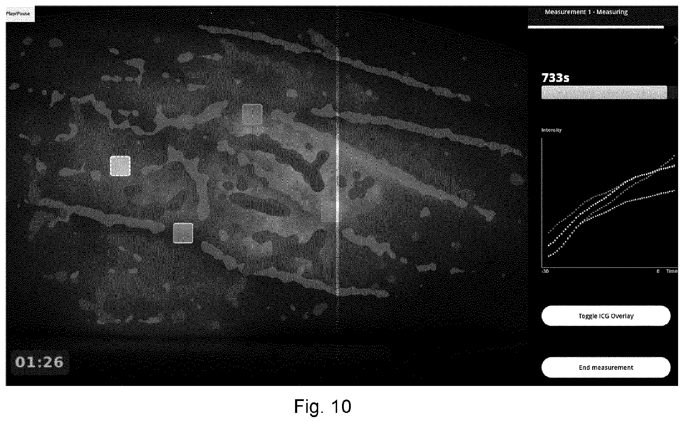

Fig. 10 shows an image where the arteries and veins shown in figs. 8-9 are visually enhanced in red (arteries) and blue (veins) and superimposed in an image such that arteries and veins are clearly visible and easily distinguishable. This is an example of an Augmented Reality (AR) view which can be offered to the surgeon during a medical procedure employing the presently disclosed approach. The red pixels have been mapped to the artery-group and the blue pixels have been mapped to the veins-group. These mappings can be continuously updated because the micro-bolus regime can run in the background. In practice a given group, such as the artery-group, has a known phase which can be identified at an initial learning period of the micro-bolus regime where the artery phase can be associated with the time point of each bolus injection. Once the artery phase is known a segment, e.g. a few seconds, of the fluorescent signal is sufficient to assess relevant perfusion curves for one or more pixels or ROIs in a sequence of images and compute a phase matching score. If the phase in the pixels / ROIs matches with the artery phase, the phase matching score will be high, and the pixels can consequently be identified as arteries and in this case colored with red. Similar calculations can be performed for the veins. Such phase matching assessments can be provided both during inflow of ICG and during the washout period. The presently disclosed approach of continuously identifying blood vessels in tissue can therefore run in the background continuously.

## Claims

1. A computer program having instructions which, when executed by a computing device or computing system, cause the computing device or computing system to carry out a method for identifying blood vessels in tissue of a subject, e.g. during a medical procedure, the method comprising the steps of
- continuously acquiring fluorescence images of the tissue wherein a fluorescent signal is oscillating with a predetermined pattern, the pattern generated from a series of boluses of at least one fluorescent imaging agent, and wherein the series of boluses is administered with a predefined and/or controlled duration between subsequent boluses determining the pattern, and
- analysing at least part of the fluorescence images,
- determining, during analysing, at least one time difference selected from the group of:
• time difference between bolus injection and artery fluorescent signal or vein fluorescent signal,
• time difference between artery fluorescent signal and vein fluorescent signal,
• time difference between artery fluorescent signal or vein fluorescent signal and tissue fluorescent signal, and
- continuously identifying blood vessels in the fluorescence images of the tissue based on said time difference(s) and the fluorescent signal oscillating with the predetermined pattern.

2. The computer program of claim 1, wherein the predetermined pattern has a frequency determined by the predefined and/or controlled duration between subsequent boluses.

3. The computer program of any of the preceding claims, wherein the identified blood vessels are combined to identify one or more networks of interconnected blood vessels, and/or
wherein the pattern comprises a period of between 1 and 5 minutes over a time period of at least 15 minutes and wherein the fluorescent signal is oscillating in intensity in accordance with this predetermined pattern,
and/or
wherein the identified blood vessels are continuously displayed and visualized on a screen.

4. The computer program of any of the preceding claims, comprising the step of determining a time difference between artery fluorescent signal and vein fluorescent signal, and distinguishing arteries and veins in the identified blood vessels based on said time difference and the predetermined pattern of the oscillating fluorescent signal, wherein the identified arteries and veins preferably are superimposed into white light images, visually enhanced such that arteries and veins are visually distinguishable and displayed on a screen.

5. The computer program of any of the preceding claims, wherein a sequence of the acquired fluorescence images are analysed and pixels in the fluorescence images are classified as either 1) artery, 2) vein, 3) surrounding tissue, or 4) otherwise, based on the phase of the fluorescent signal in the respective pixels relative to the predetermined oscillating pattern,
and/or
wherein blood vessels are identified and visualized by image filtering, such as edge filtering.

6. The computer program of any of the preceding claims, wherein a series of boluses of at least one fluorescent imaging agent is provided into a vein of the subject during the image acquisition thereby generating the predetermined pattern of oscillating fluorescent intensity, and wherein the series of boluses is administered with a predefined duration between subsequent boluses.

7. The computer program of claim 6, wherein the fluorescent imaging agent is ICG and wherein each bolus of ICG corresponds to less than 0.01 mg ICG/kg body weight,
or
wherein the fluorescent imaging agent is ICG and wherein each bolus of ICG corresponds to less than 0.005 mg ICG/kg body weight,
or
wherein the fluorescent imaging agent is ICG and wherein each bolus of ICG corresponds to less than 0.004 mg ICG/kg body weight, more preferably less than 0.003 mg ICG/kg body weight, even more preferably less than 0.002 mg ICG/kg body weight, most preferably less than 0.001 mg ICG/kg body weight.

8. The computer program of any of the preceding claims, wherein the series of boluses is injected automatically by a controllable injection pump.

9. The computer program according to any of the preceding claims, wherein the imaged tissue is part of an anatomical structure in the gastrointestinal tract, preferably selected from the buccal cavity; pharynx; the small intestine including duodenum, jejunum, and ileum; the stomach, including esophagus, cardia, and pylorus; the large intestine including cecum, colon, rectum and the anal canal, and/or
wherein the imaged tissue is subject to peristaltic movement during the medical procedure,
and/or
wherein the imaged tissue is part of an internal organ of the subject, or part of the skin of the subject, or part of a wound of the subject.

10. The computer program of any of the preceding claims, wherein the images are acquired during thyroid surgery and wherein blood vessels in one or more of the parathyroid glands are identified and visualized to medical personnel involved in the surgery.

11. The computer program according to any of the preceding claims, wherein the at least one fluorescence imaging agent is selected from the group of: indocyanine green (ICG), fluorescein isothiocyanate, rhodamine, phycoerythrin, phycocyanin, allophycocyanin, ophthaldehyde, fluorescamine, rose Bengal, trypan blue, fluoro-gold, green fluorescence protein, a flavin, methylene blue, porphysomes, cyanine dye, IRDDye800CW, CLR 1502 combined with a targeting ligand, OTL38 combined with a targeting ligand, or a combination thereof.

12. A system comprising at least one processing unit, or having access to a processing unit, and memory having instructions stored thereon, instructions, when executed by the processing unit(s), performs a method for identifying blood vessels in tissue of a subject, the method comprising the steps of:
- continuously generating a fluorescent signal from blood vessels in the tissue wherein the fluorescent signal is oscillating with a predetermined pattern, the pattern generated from a series of boluses of at least one fluorescent imaging agent, and wherein the series of boluses is administered with a predefined and/or controlled duration between subsequent boluses determining the pattern,,
- continuously receiving fluorescence images of the tissue
- analysing at least part of the fluorescence images and determining at least one time difference selected from the group of:
• time difference between bolus injection and artery fluorescent signal or vein fluorescent signal,
• time difference between artery fluorescent signal and vein fluorescent signal,
• time difference between artery fluorescent signal or vein fluorescent signal and tissue fluorescent signal, and
- continuously identifying blood vessels in the fluorescence images of the tissue based on said time difference(s) and the fluorescent signal oscillating with the predetermined pattern.

13. The system according to claim 12, comprising a controllable injection pump for holding at least one first fluorescence imaging agent, the injection pump being configured for injecting a series of predefined boluses of said first fluorescence imaging agent into a vein of the subject, thereby generating the fluorescent signal which is oscillating with the predetermined pattern,
and/or
wherein the fluorescence agent is ICG and wherein the amount of ICG in a predefined bolus is less than 0.005 mg /kg body weight and wherein the system is configured to inject boluses with an interval of between 1 and 5 minutes.

14. The system according to any of claims 12-13, wherein the method comprise steps of any of claims 2-11

## Patentansprüche

1. Computerprogramm mit Anweisungen, die, wenn sie von einer Rechenvorrichtung oder einem Rechensystem ausgeführt werden, die Rechenvorrichtung oder das Rechensystem veranlassen, ein Verfahren zum Identifizieren von Blutgefäßen im Gewebe eines Subjekts, z. B. während einer medizinischen Prozedur, auszuführen, das Verfahren umfassend die folgenden Schritte kontinuierliches Aufnehmen von Fluoreszenzbildern des Gewebes, wobei ein Fluoreszenzsignal mit einem vorbestimmten Muster oszilliert, wobei das Muster aus einer Reihe von Boli mindestens eines fluoreszierenden bildgebenden Mittels erzeugt wird, und wobei die Reihe von Boli mit einer vordefinierten und/oder gesteuerten Dauer zwischen aufeinanderfolgenden Boli verabreicht wird, die das Muster bestimmen, und
Analysieren von mindestens einem Teil der Fluoreszenzbilder,
Bestimmen mindestens eines Zeitunterschieds während des Analysierens, ausgewählt aus der Gruppe von:
• Zeitdifferenz zwischen Bolusinjektion und Arterienfluoreszenzsignal oder Venenfluoreszenzsignal,
• Zeitdifferenz zwischen dem Arterienfluoreszenzsignal und dem Venenfluoreszenzsignal,
• Zeitdifferenz zwischen dem Arterienfluoreszenzsignal und dem Venenfluoreszenzsignal und Gewebefluoreszenzsignal und
kontinuierliches Identifizieren von Blutgefäßen in den Fluoreszenzbildern des Gewebes basierend auf der/den Zeitdifferenz(en) und dem Fluoreszenzsignal, das mit dem vorbestimmten Muster oszilliert.

2. Computerprogramm nach Anspruch 1, wobei das vorbestimmte Muster eine Frequenz aufweist, die durch die vordefinierte und/oder gesteuerte Dauer zwischen aufeinanderfolgenden Boli bestimmt wird.

3. Computerprogramm nach einem der vorstehenden Ansprüche, wobei die identifizierten Blutgefäße kombiniert werden, um ein oder mehrere Netzwerke von miteinander verbundenen Blutgefäßen zu identifizieren, und/oder
wobei das Muster eine Periode zwischen 1 und 5 Minuten über eine Zeitperiode von mindestens 15 Minuten umfasst und wobei das Fluoreszenzsignal in seiner Intensität gemäß diesem vorbestimmten Muster oszilliert,
und/oder
wobei die identifizierten Blutgefäße kontinuierlich angezeigt und auf einem Bildschirm sichtbar gemacht werden.

4. Computerprogramm nach einem der vorstehenden Ansprüche, umfassend den Schritt des Bestimmens einer Zeitdifferenz zwischen dem Arterienfluoreszenzsignal und dem Venenfluoreszenzsignal und des Unterscheidens von Arterien und Venen in den identifizierten Blutgefäßen basierend auf dieser Zeitdifferenz und dem vorbestimmten Muster des oszillierenden Fluoreszenzsignals, wobei die identifizierten Arterien und Venen vorzugsweise in Weißlichtbildern überlagert, visuell verbessert, sodass Arterien und Venen visuell unterscheidbar sind, und auf einem Bildschirm angezeigt werden.

5. Computerprogramm nach einem der vorstehenden Ansprüche, wobei eine Sequenz der aufgenommenen Fluoreszenzbilder analysiert wird und Pixel in den Fluoreszenzbildern entweder als 1) Arterie, 2) Vene, 3) umgebendes Gewebe oder 4) anderweitig klassifiziert werden, basierend auf der Phase des Fluoreszenzsignals in den jeweiligen Pixeln relativ zu dem vorbestimmten oszillierenden Muster,
und/oder
wobei Blutgefäße durch Bildfilterung, wie beispielsweise durch Kantenfilterung, identifiziert und sichtbar gemacht werden.

6. Computerprogramm nach einem der vorstehenden Ansprüche, wobei eine Reihe von Boli mindestens eines fluoreszierenden bildgebenden Mittels in eine Vene des Subjekts während der Bildaufnahme bereitgestellt wird, wodurch das vorbestimmte Muster der oszillierenden Fluoreszenzintensität erzeugt wird, und wobei die Reihe von Boli mit einer vordefinierten Dauer zwischen aufeinanderfolgenden Boli verabreicht wird.

7. Computerprogramm nach Anspruch 6, wobei das fluoreszierende bildgebende Mittel ICG ist und wobei jeder Bolus von ICG weniger als 0,01 mg ICG/kg Körpergewicht entspricht,
oder
wobei das fluoreszierende bildgebende Mittel ICG ist und wobei jeder Bolus von ICG weniger als 0,005 mg ICG/kg Körpergewicht entspricht,
oder
wobei das fluoreszierende bildgebende Mittel ICG ist und wobei jeder Bolus von ICG weniger als 0,004 mg ICG/kg Körpergewicht, bevorzugter weniger als 0,003 mg ICG/kg Körpergewicht, noch bevorzugter weniger als 0,002 mg ICG/kg Körpergewicht, am meisten bevorzugt weniger als 0,001 mg ICG/kg Körpergewicht entspricht.

8. Computerprogramm nach einem der vorstehenden Ansprüche, wobei die Serie von Boli automatisch durch eine steuerbare Injektionspumpe injiziert wird.

9. Computerprogramm nach einem der vorstehenden Ansprüche, wobei das abgebildete Gewebe Teil einer anatomischen Struktur im Magendarmtrakt ist, vorzugsweise ausgewählt aus der Mundhöhle, dem Rachen, dem Dünndarm einschließlich Zwölffingerdarm, Jejunum und Ileum, dem Magen einschließlich Speiseröhre, Kardia und Pylorus, dem Dickdarm einschließlich Zökum, Kolon, Rektum und dem Analkanal,
und/oder
wobei das abgebildete Gewebe während der medizinischen Prozedur einer peristaltischen Bewegung unterworfen ist,
und/oder
wobei das abgebildete Gewebe ein Teil eines inneren Organs des Subjekts oder ein Teil der Haut des Subjekts oder ein Teil einer Wunde des Subjekts ist.

10. Computerprogramm nach einem der vorstehenden Ansprüche, wobei die Bilder während einer Schilddrüsenoperation aufgenommen werden und wobei Blutgefäße in einer oder mehreren der Nebenschilddrüsen identifiziert und für das an der Operation beteiligte medizinische Personal sichtbar gemacht werden.

11. Computerprogramm nach einem der vorstehenden Ansprüche, wobei das mindestens eine fluoreszierende bildgebende Mittel ausgewählt ist aus der Gruppe von: Indocyaningrün (ICG), Fluoresceinisothiocyanat, Rhodamin, Phycoerythrin, Phycocyanin, Allophycocyanin, Ophthaldehyd, Fluorescamin, Rose Bengal, Trypanblau, Fluorgold, grünem Fluoreszenzprotein, einem Flavin, Methylenblau, Porphysomen, Cyaninfarbstoff, IRDDye800CW, CLR 1502 in Kombination mit einem Targeting-Liganden, OTL38 in Kombination mit einem Targeting-Liganden oder einer Kombination davon.

12. System, umfassend mindestens eine Verarbeitungseinheit oder mit Zugriff auf eine Verarbeitungseinheit und einen Speicher mit darauf gespeicherten Anweisungen, wobei die Anweisungen, wenn sie von der/den Verarbeitungseinheit(en) ausgeführt werden, ein Verfahren zum Identifizieren von Blutgefäßen im Gewebe eines Subjekts durchführen, das Verfahren umfassend die folgenden Schritte:
kontinuierliches Erzeugen eines Fluoreszenzsignals von Blutgefäßen im Gewebe, wobei das Fluoreszenzsignal mit einem vorbestimmten Muster oszilliert, wobei das Muster aus einer Reihe von Boli mindestens eines fluoreszierenden bildgebenden Mittels erzeugt wird, und wobei die Reihe von Boli mit einer vordefinierten und/oder gesteuerten Dauer zwischen aufeinanderfolgenden Boli, die das Muster bestimmen, verabreicht wird,
kontinuierliches Empfangen von Fluoreszenzbildern des Gewebes Analysieren mindestens eines Teils der Fluoreszenzbilder und Bestimmen mindestens einer Zeitdifferenz, ausgewählt aus der Gruppe von:
• Zeitdifferenz zwischen Bolusinjektion und Arterienfluoreszenzsignal oder Venenfluoreszenzsignal,
• Zeitdifferenz zwischen dem Arterienfluoreszenzsignal und dem Venenfluoreszenzsignal,
• Zeitdifferenz zwischen Arterien- oder Venenfluoreszenzsignal und Gewebefluoreszenzsignal und
kontinuierliches Identifizieren von Blutgefäßen in den Fluoreszenzbildern des Gewebes basierend auf der/den Zeitdifferenz(en) und dem Fluoreszenzsignal, das mit dem vorbestimmten Muster oszilliert.

13. System nach Anspruch 12, umfassend eine steuerbare Injektionspumpe zur Aufnahme mindestens eines ersten fluoreszierenden bildgebenden Mittels, wobei die Injektionspumpe konfiguriert ist, um eine Reihe von vordefinierten Boli des ersten fluoreszierenden bildgebenden Mittels in eine Vene des Subjekts zu injizieren, wodurch das fluoreszierende Signal erzeugt wird, das mit dem vorbestimmten Muster oszilliert,
und/oder
wobei das Fluoreszenzmittel ICG ist und wobei die Menge an ICG in einem vordefinierten Bolus weniger als 0,005 mg/kg Körpergewicht beträgt und wobei das System konfiguriert ist, um Boli mit einem Intervall von zwischen 1 und 5 Minuten zu injizieren.

14. System nach einem der Ansprüche 12 bis 13, wobei das Verfahren Schritte nach einem der Ansprüche 2 bis 11 umfasst

## Revendications

1. Programme informatique ayant des instructions qui, lorsqu'elles sont exécutées par un dispositif informatique ou un système informatique, amènent le dispositif informatique ou le système informatique à mettre en œuvre un procédé pour l'identification de vaisseaux sanguins dans les tissus d'un sujet, par exemple lors d'une intervention médicale, ce procédé comprenant les étapes suivantes et
- l'acquisition continue d'images par fluorescence des tissus dans lequel un signal fluorescent oscille selon un motif prédéterminé, le motif étant généré à partir d'une série de bolus d'au moins un agent d'imagerie fluorescente, et dans lequel la série de bolus est administrée selon une durée prédéfinie et/ou commandée entre les bolus successifs déterminant le motif, et
- l'analyse d'au moins une partie des images par fluorescence,
- la détermination, lors de l'analyse, d'au moins une différence de temps sélectionnée à partir du groupe suivant :
• la différence de temps entre l'injection du bolus et le signal fluorescent artériel ou le signal fluorescent veineux,
• la différence de temps entre le signal fluorescent artériel et le signal fluorescent veineux,
• la différence de temps entre le signal fluorescent artériel ou le signal fluorescent veineux et le signal fluorescent des tissus, et
- l'identification continue de vaisseaux sanguins dans les images par fluorescence du tissu sur la base de ladite ou desdites différences de temps et du signal fluorescent oscillant selon le modèle prédéterminé.

2. Programme informatique selon la revendication 1, dans lequel le modèle prédéterminé a une fréquence déterminée par la durée prédéfinie et/ou commandée entre des bolus successifs.

3. Programme informatique selon l'une quelconque des revendications précédentes, dans lequel les vaisseaux sanguins identifiés sont combinés pour identifier un ou plusieurs réseaux de vaisseaux sanguins interconnectés, et/ou
dans lequel le motif comprend une période comprise entre 1 et 5 minutes sur une période d'au moins 15 minutes et dans lequel le signal fluorescent oscille en intensité selon ce motif prédéterminé,
et/ou
dans lequel les vaisseaux sanguins identifiés sont affichés et visualisés en continu sur un écran.

4. Programme informatique selon l'une quelconque des revendications précédentes, comprenant l'étape de détermination d'une différence de temps entre le signal fluorescent artériel et le signal fluorescent veineux, et de distinction des artères et des veines dans les vaisseaux sanguins identifiés sur la base de ladite différence de temps et du modèle prédéterminé du signal fluorescent oscillant, dans lequel les artères et les veines identifiées sont de préférence superposées à des images en lumière blanche, visuellement améliorées de sorte que les artères et les veines soient visuellement distinguables et affichées sur un écran.

5. Le programme informatique selon l'une quelconque des revendications précédentes, dans lequel une séquence d'images par fluorescence acquises est analysée et des pixels des images par fluorescence sont classés comme 1) artère, 2) veine, 3) tissu environnant ou 4) autre, sur la base de la phase du signal fluorescent dans les pixels respectifs par rapport au motif oscillant prédéterminé,
et/ou
dans lequel des vaisseaux sanguins sont identifiés et visualisés par filtrage d'image, tel que le filtrage des contours.

6. Programme informatique selon l'une quelconque des revendications précédentes, dans lequel une série de bolus d'au moins un agent d'imagerie fluorescente est administrée dans une veine du sujet pendant l'acquisition de l'image, générant ainsi le modèle prédéterminé d'intensité fluorescente oscillante, et dans lequel la série de bolus est administrée avec une durée prédéfinie entre les bolus successifs.

7. Programme informatique selon la revendication 6, dans lequel l'agent d'imagerie fluorescente est l'ICG et dans lequel chaque bolus d'ICG correspond à moins de 0,01 mg d'ICG/kg de poids corporel,
ou
dans lequel l'agent d'imagerie fluorescente est l'ICG et dans lequel chaque bolus d'ICG correspond à moins de 0,005 mg d'ICG/kg de poids corporel,
ou
dans lequel l'agent d'imagerie fluorescente est l'ICG et dans lequel chaque bolus d'ICG correspond à moins de 0,004 mg d'ICG/kg de poids corporel, de préférence à moins de 0,003 mg d'ICG/kg de poids corporel, de préférence davantage à moins de 0,002 mg d'ICG/kg de poids corporel, et encore de préférence à moins de 0,001 mg d'ICG/kg de poids corporel.

8. Programme informatique selon l'une quelconque des revendications précédentes, dans lequel la série de bolus est injectée automatiquement par une pompe d'injection commandable.

9. Programme informatique selon l'une quelconque des revendications précédentes, dans lequel les tissus imagés font partie d'une structure anatomique dans le tube digestif, de préférence choisie dans la cavité buccale ; le pharynx ; l'intestin grêle, comportant le duodénum, le jéjunum et l'iléon ; l'estomac, comportant l'œsophage, le cardia et le pylore ; le gros intestin, comportant le cæcum, le côlon, le rectum et le canal anal,
et/ou
dans lequel le tissu imagé est soumis à un mouvement péristaltique au cours de l'intervention médicale,
et/ou
dans lequel le tissu imagé fait partie d'un organe interne du sujet, ou d'une partie de la peau du sujet, ou d'une partie d'une plaie du sujet.

10. Programme informatique selon l'une quelconque des revendications précédentes, dans lequel les images sont acquises pendant une chirurgie thyroïdienne et dans lequel les vaisseaux sanguins de l'une ou plusieurs de glandes parathyroïdes sont identifiés et visualisés pour le personnel médical participant à la chirurgie.

11. Programme informatique selon l'une quelconque des revendications précédentes, dans lequel au moins un agent d'imagerie par fluorescence est choisi parmi les suivants : vert d'indocyanine (ICG), isothiocyanate de fluorescéine, rhodamine, phycoérythrine, phycocyanine, allophycocyanine, ophtalaldéhyde, fluorescamine, rose bengale, bleu trypan, or fluorescent, protéine fluorescente verte, flavine, bleu de méthylène, porphysomes, colorant cyanine, IRDDye800CW, CLR 1502 combiné à un ligand de ciblage, OTL38 combiné à un ligand de ciblage, ou une combinaison de ceux-ci.

12. Système comprenant au moins une unité de traitement, ou ayant accès à une unité de traitement, et une mémoire ayant des instructions stockées sur celle-ci, des instructions qui lorsqu'elles sont exécutées par la ou les unité(s) de traitement, réalisent un procédé pour l'identification de vaisseaux sanguins dans les tissus d'un sujet, le procédé comprenant les étapes suivantes :
- la génération continue d'un signal fluorescent à partir des vaisseaux sanguins dans les tissus dans lequel le signal fluorescent oscille selon un motif prédéterminé, le motif étant généré à partir d'une série de bolus d'au moins un agent d'imagerie fluorescente, et dans lequel la série de bolus est administrée selon une durée prédéfinie et/ou commandée entre les bolus successifs déterminant le motif,
- la réception continue d'images par fluorescence des tissus
- l'analyse d'au moins une partie des images par fluorescence et la détermination d'au moins une différence de temps sélectionnée à partir du groupe suivant :
• la différence de temps entre l'injection du bolus et le signal fluorescent artériel ou le signal fluorescent veineux,
• la différence de temps entre le signal fluorescent artériel et le signal fluorescent veineux,
• la différence de temps entre le signal fluorescent artériel ou le signal fluorescent veineux et le signal fluorescent des tissus, et
- l'identification continue de vaisseaux sanguins dans les images par fluorescence du tissu sur la base de ladite ou desdites différences de temps et du signal fluorescent oscillant selon le modèle prédéterminé.

13. Système selon la revendication 12, comprenant une pompe d'injection commandable pour contenir au moins un premier agent d'imagerie par fluorescence, la pompe d'injection étant configurée pour l'injection d'une série de bolus prédéfinis dudit premier agent d'imagerie par fluorescence dans une veine du sujet, générant ainsi le signal fluorescent oscillant selon le modèle prédéterminé,
et/ou
dans lequel l'agent par fluorescence est l'ICG et dans lequel la quantité d'ICG dans un bolus prédéfini est inférieure à 0,005 mg/kg de poids corporel et dans lequel le système est configuré pour injecter des bolus à un intervalle compris entre 1 et 5 minutes.

14. Système selon l'une quelconque des revendications 12 et 13, dans lequel le procédé comprend les étapes de l'une quelconque des revendications 2 à 11.
